(19) 

Europäisches
Patentamt
European
Patent Office
Office européen
des brevets

(11)  **EP 4 570 280 A1**

(12) **EUROPEAN PATENT APPLICATION**
published in accordance with Art. 153(4) EPC

(43) Date of publication:
**18.06.2025 Bulletin 2025/25**

(21) Application number: **23872651.7**

(22) Date of filing: **02.10.2023**

(51) International Patent Classification (IPC):
**A61M 1/36** $^{(2006.01)}$

(52) Cooperative Patent Classification (CPC):
**A61M 1/36**

(86) International application number:
**PCT/JP2023/035892**

(87) International publication number:
**WO 2024/071432 (04.04.2024 Gazette 2024/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.09.2022 JP 2022158212**

(71) Applicant: **NBC Meshtec Inc.
Hino-shi, Tokyo 191-0053 (JP)**

(72) Inventors:
• **TAKIGUCHI, Makoto
Hino-shi, Tokyo 191-0053 (JP)**
• **YAMAGUCHI, Masayuki
Hino-shi, Tokyo 191-0053 (JP)**
• **KOSAKA, Torino
Hino-shi, Tokyo 191-0053 (JP)**
• **MOTOJIMA, Nobukazu
Hino-shi, Tokyo 191-0053 (JP)**

(74) Representative: **J A Kemp LLP
80 Turnmill Street
London EC1M 5QU (GB)**

(54) **HEMOFILTRATION FILTER AND HEMOFILTRATION FILTER MEMBER**

(57) [Problem] To provide a hemofiltration filter that is capable of removing foreign substances such as blood clots and proteins while maintaining a sufficient blood filtration flow rate.

[Solution] A mesh-type hemofiltration filter formed of resin fibers, characterized in that: the mesh number is 300 mesh or more; the aperture ratio is 30-50% inclusive; the fiber diameter of the fibers is 10-40 $\mu$m inclusive; and the weave pattern is a plain weave or a triple twill diagonal weave.

EP 4 570 280 A1

**Description**

Technical Field

The present invention relates to a blood filtration filter, for example, for hemodialysis apparatuses and heart-lung machines.

Background Art

Blood filtration filters for removing thrombi and protein substances generated during the circulation of a patient's blood are included, for example, in heart-lung machines used in treatment of respiratory failure due to COVID-19, pneumonia, and the like, as well as in hemodialysis apparatuses for blood purification, for example, in treatment of renal failure. The treatments often last for a long period of time. If biological components such as blood or body fluid contact with the surfaces of materials used in medical devices, the materials are recognized as foreign substances. This can lead to adhesion of platelets or proteins, a deterioration in performance of the materials, and, in addition, serious problems such as the occurrence of biological reactions and the formation of thrombi. In order to solve these, a technique has been disclosed that involves coating the surface of a blood circuit, which comes into contact with blood, with heparin or a hydrophilic polymer to improve blood compatibility and prevent the generation of thrombi (Patent Document 1). Additionally, a method has been disclosed that involves modifying the surface of a blood circuit with a fluorine-containing polymer to make the surface antithrombotic, thereby reducing the amount of anticoagulants used (Patent Document 2).

Prior Art Document

Patent Document

Patent Document 1: Japanese Unexamined Patent Application Publication No. 2004-8693
Patent Document 2: Japanese Unexamined Patent Application Publication No. 2016-52525

Summary of the Invention

Technical Problem to be Solved by the Invention

The blood filtration flow rate through a blood filtration filter is exemplified as another cause for the generation of thrombi. It is known that a low blood filtration flow rate through the blood filtration filter leads to a turbulent blood flow to increase stagnant blood, resulting in thrombi. Therefore, it is considered to increase the aperture ratio of the filter for enhancing the blood filtration flow rate through the blood filtration filter. However, an excessive increase in aperture ratio may allow thrombi and protein substances, which are intended to be removed, to pass through. Accordingly, the present invention is directed to providing a blood filtration filter that enables removing foreign substances such as thrombi and protein substances while sufficiently maintaining the blood filtration flow rate.

Means for Solving the Solution to Problem

That is, means for solving the above-mentioned problem is as follows.

(1) A mesh-type blood filtration filter formed from resin fibers, wherein the filter has a mesh count of 300 or more, the filter has an aperture ratio of 30% or more and 50% or less, the fibers have a diameter of 10 $\mu$m or more and 40 $\mu$m or less, and the weave structure is a plain weave or a 2/1 twill weave.
(2) The blood filtration filter according to the above-mentioned (1), wherein the filter has an aperture size in the warp direction with a standard deviation of less than 3 $\mu$m and an aperture size in the weft direction with a standard deviation of less than 3 $\mu$m.
(3) The blood filtration filter according to the above-mentioned (1), wherein the filter is formed of monofilament fibers.
(4) The blood filtration filter according to the above-mentioned (1), wherein OPs (openings) of the filter are 20 $\mu$m or more and 50 $\mu$m or less.
(5) A blood filtration filter component, comprising the blood filtration filter according to any one of the above-mentioned (1) to (4) and a supporting member holding the filter.

Advantageous Effect of the Invention

According to the present invention, a blood filtration filter that enables removing foreign substances such as thrombi and protein substances while sufficiently maintaining the blood filtration flow rate can be provided.

Mode for Carrying Out the Invention

A blood filtration filter of the present embodiment will be described in detail below. The blood filtration filter of the present embodiment is a mesh-type filter woven from threads (fibers). The blood filtration filter functions as a filter for removing foreign substances such as thrombi while passing blood through the aperture portions of the weave structure.

The mesh count as used herein means the number of weft threads or warp threads per 25.4 mm (1 inch) length parallel to the warp or the weft of the fabric forming the blood filtration filter (threads/inch).

The aperture ratio is an index for indicating the area ratio of the aperture portions of a mesh fabric and can be determined in accordance with the following equation (1). In the present embodiment, the aperture ratio is determined as follows: measure OP and thread diameter at twenty random points each in both the warp direction and the weft direction, for example, using a digital microscope; calculate the average values from the actual measurements; define these average values as the warp direction OP, the weft direction OP, the warp diameter, and the weft diameter; substitute these values into the following equation (1) to determine the aperture ratio. The thread diameters may be measured by measuring the diameters of the threads surrounding the positions where the OPs are measured.

$$\text{Aperture ratio (\%)} =$$
$$(\text{warp direction OP} \times \text{weft direction OP})/((\text{warp direction OP} + \text{weft diameter}) \times (\text{weft direction OP} + \text{warp diameter})) \times 100 \qquad (1)$$

The OP (opening) is the distance between threads. The warp direction OP is the distance between weft threads. The weft direction OP is the distance between warp threads. In the present embodiment, the OPs are values actually measured through a digital microscope. The thread diameters can also be measured through the digital microscope. Since threads are flattened and thickened at points of intersection, the OPs and the thread diameters should be measured at positions at which the threads are unflattened between the points of intersection. If the warp threads cannot be distinguished from the weft threads, any threads may be assumed to be the warp, and the others may be assumed to be the weft. In this case, the distances between the weft threads may be defined as warp direction OPs, and the distances between the warp threads may be defined as weft direction OPs.

The "aperture" and "aperture portion" as used herein refer to the space of the mesh (not including threads).

First, the blood filtration filter of the present embodiment is characterized in that the fiber diameter (thread diameter) of the warp and weft threads constituting the fabric (mesh) is 10 μm or more and 40 μm or less. Although it is desirable to weave a fabric having a higher aperture ratio for increasing the blood filtration flow rate, as mentioned above, only an increase in the aperture ratio does not enable removing foreign substances such as thrombi. In order to remove foreign substances, the mesh count has been increased to as large as 300 or more to reduce the OP of the fabric, and fibers with a small fiber diameter of 10 μm or more and 40 μm or less have been used to develop a blood filtration filter with a high aperture ratio in the present embodiment. Fibers having a fiber diameter of less than 10 μm lack strength, resulting in tears in the filter. Fibers having a fiber diameter of more than 40 μm make it difficult to manufacture fabric having a high aperture ratio described later. It is preferable to use fibers having the same fiber diameter as the warp and the weft.

As mentioned above, the mesh count is 300 or more in the blood filtration filter of the present embodiment. The mesh count in the warp direction, in which the warp threads extend, and the mesh count in the weft direction, in which the weft threads extend, may be the same or different. Even if the mesh counts are different, both mesh counts are 300 or more. As mentioned above, if the mesh count is 300 or more, the fibers having the fiber diameter of the present embodiment enable reducing the OPs of the filter sufficiently and removing foreign substances such as thrombi. If the mesh count is less than 300, the blood filtration filter does not enable removing foreign substances sufficiently. The upper limit of the mesh count of the filter of the present embodiment may be any number. However, considering the current weaving technology and the diameter of available synthetic fibers, it is considered that the mesh count may be limited to 800, so the upper limit of the mesh count can be 800 or less.

The blood filtration filter preferably has an OP of 20 μm or more and 50 μm or less for the following reason: An OP of less than 20 μm may remove essential components such as red blood cells, and an OP of more than 50 μm does not

enable removing foreign substances such as thrombi. The warp direction OP and the weft direction OP may have the same size or different sizes. The OP is more preferably 40 $\mu$m or less. An OP of 40 $\mu$m or less enables removing foreign substances more sufficiently.

Resin fibers can be used as the fibers constituting the blood filtration filter of the present embodiment. Any resin can be used as the fibers for the blood filtration filters, as long as it can be molded into a fiber. The user can choose the appropriate resin based on their needs. For example, thermoplastic resin or thermoplastic elastomer is usable as the resin. Specific examples include polyethylene resin; polypropylene resin; polystyrene resin; ABS resin; AS resin; EVA resin; polymethylpentene resin; polyvinyl chloride resin; polyvinylidene chloride resin; polymethyl acrylate resin; polyvinyl acetate resin; polyamide resin; polyimide resin; polycarbonate resin; polyester resins such as polyethylene terephthalate resin (PET) and polybutylene terephthalate resin; thermoplastic resins such as polyacetal resin, polyarylate resin, polysulfone resin, polyphenylene sulfide (PPS), and liquid crystal polymer(LCP); silicone resin; styrene-based elastomer such as polystyrene elastomer; olefinic elastomers such as polyethylene elastomer and polypropylene elastomer; polyurethane-based elastomer such as polyurethane elastomer; and thermoplastic elastomers such as vinyl chloride-based elastomer, polyester-based elastomer, and nylon-based elastomer.

The fibers constituting the blood filtration filter of the present embodiment may be either monofilament or multifilament. In order to obtain a high aperture ratio, it is preferable that the fibers be monofilament fibers, which are less likely to be flattened to reduce the aperture ratio.

The blood filtration filter of the present embodiment is a woven fabric with an aperture ratio of 30% or more and 50% or less. If the aperture ratio is less than 30%, blood that cannot pass through the filter stagnates near the inlet of the filter, resulting in poor blood flow. Consequently, blood contacts with a medical device upstream or downstream of the blood filtration filter for a longer period of time, so that thrombi are likely to be formed. If the thrombi are formed, the thrombi are likely to clog the filter, resulting in a further decrease in the blood filtration flow rate. For example, the reduced flow rate may aggravate the physical condition of the patient. An aperture ratio of more than 50% increases the misalignment of filter threads to make the OPs nonuniform, or deteriorates the strength of the filter, leading to tears. Such an aperture ratio over 50% is not, therefore, preferable. Based on the relationship between the stability of the fabric and the blood filtration flow rate, it is preferable to adjust the aperture ratio to 40% or more and 45% or less.

Furthermore, the blood filtration filter of the present embodiment is characterized in that the weave structure thereof is a plain weave or a 2/1 twill weave ("mitsu-aya shamon-ori"; weaving 2:1 twill). A plain weave is a woven fabric woven by crossing the warp and the weft one by one alternately. A shamon-ori (twill weave), which is also referred to as aya-ori (twill weave), is a woven fabric in which each warp thread passes over two or three weft threads and then passes under one weft thread repeatedly. A twill weave in which each warp thread passes over two weft threads and then passes under one weft thread is referred to as "mitsu-aya (2/1 twill)" and is also referred to as a 2/1 shamon-ori (2/1 twill weave) or 2/1 aya-ori (2/1 twill weave). A 1/2 shamon-ori (1/2 twill weave), in which each weft thread passes over two warp threads and then passes under one warp thread, is also a twill weave of 2/1 twill, having weave structure similar thereto. In the present embodiment, a twill weave of mitsu-aya (2/1 twill) is described as a 2/1 twill weave.

In order to suppress the generation of foreign substances such as thrombi, the blood filtration filter is required to have the function of allowing blood to pass smoothly and without variation throughout the entire area of the filter through which the blood flows, besides the blood filtration flow rate. To ensure smooth passage of blood throughout the entire area of the filter, the fabric needs to have low variation of OPs. If the OPs have some variation, the pressure loss varies depending on the position of the filter. This can cause blood stagnation, which leads to thrombi. The thread slippage (misalignment) does not often make the OPs uneven in the weave structure of fabric woven using fibers having ordinary thickness. However, since the blood filtration filter of the present embodiment is a fabric woven using fine fibers and having a high aperture ratio, the filter is very coarse, so that the warp and the weft may misalign during weaving (which is referred to as "misalignment" hereinafter). This misalignment makes the OPs nonuniform.

The blood filtration filter of the present embodiment has the weave structure that is a plain weave or a 2/1 twill weave (including both a 2/1 twill weave and a 1/2 twill weave). This structure suppresses the above-mentioned misalignment to reduce the variation of the OPs further, and allows blood to pass smoothly throughout the entire filter. Meanwhile, there are other weave structures, such as shamon-ori (twill weaves) in which the number of crossing threads is varied (twill weaves of "yotsu-aya" including a 2/2 twill weave and a 3/1 twill weave) and a shusu-ori (satin weave), referred to as satin. However, since these weave structures have many weft threads passing over or under each warp thread and few points of intersection, these weave structures lead to misalignment in a fabric woven from threads having a fine diameter (fine fibers) like the present embodiment, resulting in variation of OPs. Therefore, these weave structures are unsuitable.

Additionally, in the case of a 2/2 twill weave of yotsu-aya, the variation of OPs is larger than that of a 2/1 twill weave. Furthermore, there are differences in the variation of OPs between the warp direction, in which the warp threads extend, and the weft direction, in which the weft threads extend. One of these directions tends to have more variation than the other. Since 1/3 (3/1) twill of yotsu-aya has fewer points of intersection than a plain weave or a 2/1 twill weave, the OP sizes of the 1/3 (3/1) twill tend to vary. Meanwhile, the plain weave or the 2/1 twill weave of the present embodiment has apertures with lower variation in sizes, and the difference in the variation between the warp direction OPs and the weft direction OPs is also smaller. Therefore, the plain weave and the 2/1 twill weave are preferable. Since the difference in the variation between the warp direction OPs and the weft direction OPs is smaller, this enables suppressing blood stagnation and allows blood to pass more smoothly.

The blood filtration filter of the present embodiment preferably has OPs with a standard deviation of 3 μm or less. The OPs having a standard deviation of 3 μm or less enables reducing variation of the OPs sufficiently, thereby allowing blood to pass smoothly. The standard deviations of the OPs in the two directions of the threads (warp direction OPs and weft direction OPs) are determined respectively, and both are preferably 3 μm or less. It is preferable that the standard deviation of the warp direction be nearly equal to the standard deviation of the weft direction. Such standard deviations enable suppressing the stagnation and allow blood to pass more smoothly.

In the present embodiment, OPs are measured at twenty randomly selected points in each of the warp and weft directions. The standard deviations of the OPs in each of the warp and weft directions are then determined from these twenty measured values. The OPs can be measured with a digital microscope.

Since the blood filtration filter of the present embodiment obtained by the above-mentioned method enables removing foreign substances such as thrombi without deteriorating the blood filtration flow rate, the blood filtration filter can be used for various medical instruments such as heart-lung machines and hemodialysis apparatuses that require blood filtration. Additionally, since the filter has the warp direction OPs and the weft direction OPs with smaller variation, it can provide a blood filtration filter that suppresses the stagnation of blood and the generation of thrombi. If the blood filtration filter of the present embodiment is used for medical instruments, it can be used as a blood filtration filter component by fixing it with a supporting member, such as a frame, that holds the blood filtration filter of the present embodiment. The supporting member may be formed of a resin material that can be used for blood filtration apparatuses and others.

Examples

The embodiments of the present invention will then be described more specifically by citing Examples. The present invention is not, however, limited exclusively to these Examples.

(Example 1)

Polyester (polyethylene terephthalate (PET)) monofilaments having a thread diameter of 19 μm were woven into a 420-mesh fabric of a plain weave as the blood filtration filter of Example 1. Twenty random warp direction OPs and twenty random weft direction OPs of the manufactured fabric were measured using a digital microscope (RH-2000, which is available from HIROX-JAPAN Co., Ltd.) to determine the standard deviation of the warp direction OPs and the standard deviation of the weft direction OPs. Twenty warp diameters and twenty weft diameters were also measured at the measurement positions of the OPs respectively. The average values of the twenty warp direction OPs, the twenty weft direction OPs, the twenty warp diameters, and the twenty weft diameters were determined and defined as the warp direction OP, the weft direction OP, the warp diameter, and the weft diameter respectively, to calculate the aperture ratio in accordance with the above-mentioned computation equation (1). Table 1 shows these results.

(Example 2)

The blood filtration filter of Example 2 was woven using the same method as in Example 1, except that the weave structure of Example 1 was changed into a 2/1 twill weave (mitsu-aya shamon-ori). The standard deviation of the warp direction OPs and the standard deviation of the weft direction OPs were determined in the same way as in Example 1 to calculate the aperture ratio. Table 1 shows these results.

(Comparative Example 1)

The blood filtration filter of Comparative Example 1 was woven using the same method as in Example 1 except that the weave structure of Example 1 was changed into a 2/2 twill weave (one of the twill weaves of yotsu-aya). The standard deviation of the warp direction OPs and the standard deviation of the weft direction OPs were determined in the same way as in Example 1 to calculate the aperture ratio. Table 1 shows these results.

(Example 3)

Liquid crystal polymer (LCP) monofilaments having a fiber diameter of 20 μm were woven into a 420-mesh fabric of a plain weave as the blood filtration filter of Example 3.

(Example 4)

PET monofilaments having a fiber diameter of 35 $\mu$m were woven into a 330-mesh fabric of a plain weave as the blood filtration filter of Example 4.

(Example 5)

PET monofilaments having a fiber diameter of 33 $\mu$m were woven into a 305-mesh fabric of a plain weave as the blood filtration filter of Example 5.

(Example 6)

PET monofilaments having a fiber diameter of 30 $\mu$m were woven into a 380-mesh fabric of a plain weave as the blood filtration filter of Example 6.

(Example 7)

Polyphenylene sulfide (PPS) monofilaments having a fiber diameter of 35 $\mu$m were woven into a 330-mesh fabric of a 1/2 twill weave as the blood filtration filter of Example 7.

(Example 8)

Polyester monofilaments having a fiber diameter of 27 $\mu$m were woven into a 315-mesh fabric of a 1/2 twill weave as the blood filtration filter of Example 8.

(Comparative Example 2)

PET monofilaments having a fiber diameter of 55 $\mu$m were woven into a 225-mesh fabric of a plain weave as the blood filtration filter of Comparative Example 2.

(Comparative Example 3)

PET monofilaments having a fiber diameter of 35 $\mu$m were woven into a 355-mesh fabric of a 1/2 twill weave as the blood filtration filter of Comparative Example 3.

Table 1 shows the manufacturing conditions for the blood filtration filters of each Example and Comparative Example. It also presents the warp direction OP and the weft direction OP (average values of the twenty OPs in each direction), the standard deviation of the warp direction OPs, the standard deviation of the weft direction OPs, and the aperture ratio of each Example and Comparative Example determined by the method shown in the above-mentioned Example 1.

[Table 1]

| | Material | Fiber diameter [$\mu$m] | Mesh count | OP [$\mu$m] | | Structure | Standard deviation [$\mu$m] | | Aperture ratio (%) |
|---|---|---|---|---|---|---|---|---|---|
| | | | | Warp direction | Weft direction | | Warp direction OP | Weft direction OP | |
| Example 1 | PET | 19 | 420 | 30.8 | 30.8 | Plain weave | 1.47 | 1.53 | 37 |
| Example 2 | PET | 19 | 420 | 33.4 | 32.5 | 2/1 Twill weave | 1.61 | 1.66 | 40 |
| Example 3 | LCP | 20 | 420 | 40.2 | 40.5 | Plain weave | 1.93 | 1.42 | 45 |
| Example 4 | PET | 35 | 330 | 43.1 | 42.4 | Plain weave | 1.46 | 1.48 | 30 |

(continued)

| | Material | Fiber diameter [μm] | Mesh count | OP [μm] | | Structure | Standard deviation [μm] | | Aperture ratio (%) |
|---|---|---|---|---|---|---|---|---|---|
| | | | | Warp direction | Weft direction | | Warp direction OP | Weft direction OP | |
| Example 5 | PET | 33 | 305 | 50.6 | 49.4 | Plain weave | 1.51 | 1.38 | 36 |
| Example 6 | PET | 30 | 380 | 37.4 | 37.1 | Plain weave | 1.31 | 1.46 | 30 |
| Example 7 | PPS | 35 | 330 | 42.5 | 42.3 | 1/2 Twill weave | 1.41 | 1.36 | 30 |
| Example 8 | PET | 27 | 315 | 53.1 | 54.2 | 1/2 Twill weave | 1.67 | 1.52 | 44 |
| Comparative Example 1 | PET | 19 | 420 | 33.7 | 32.1 | 2/2 Twill weave | 2.6 | 3.8 | 40 |
| Comparative Example 2 | PET | 55 | 225 | 58.1 | 58.4 | Plain weave | 1.55 | 1.48 | 26 |
| Comparative Example 3 | PET | 35 | 355 | 36.6 | 37.1 | 1/2 Twill weave | 2.1 | 1.67 | 26 |

The above-mentioned results show that all the samples were able to achieve an aperture ratio of 30% or more and 50% or less. The blood filtration filters of Examples 1 and 2 had smaller variation in both warp direction OPs and weft direction OPs, and less misalignment. Meanwhile, the blood filtration filter of Comparative Example 1 had weft direction OPs with larger variation than Examples 1 and 2. Although the variation of the warp direction OPs in Comparative Example 1 was smaller than that of the weft direction OPs, it was still larger than those of Examples 1 and 2. The difference in the variation between the warp direction OPs and the weft direction OPs of Comparative Example 1 was significant, and was considerably larger than those in Examples 1 and 2. It has therefore been found that the blood filtration filter of Comparative Example 1 does not satisfy the performance as a blood filtration filter.

Example 3 made of LCP as the material also had an aperture ratio of 30% or more, which resulted in a sufficient blood filtration flow rate, and also had OPs with suitable sizes, so that the filter enables removing foreign substances. Since Example 3 was a plain weave, the variation of the OPs was small.

Examples 3 to 8 also achieved an aperture ratio of 30% or more and 50% or less. The OPs had a standard deviation of less than 3 μm, and the variation of OPs was small as well.

In Comparative Example 2, the fiber diameter was greater than 40 μm and the mesh count was less than 300, so the aperture ratio was less than 30%. Since this does not enable obtaining a sufficient filtration flow rate, Comparative Example 2 is not preferable from the viewpoint of filtration efficiency. Although Comparative Example 3 satisfied the conditions such as the fiber diameter, the mesh count, and the weave structure, the filter had a low aperture ratio, and is not, therefore, preferable from the viewpoint of filtration efficiency.

**Claims**

1. A mesh-type blood filtration filter formed from resin fibers, wherein

    the filter has a mesh count of 300 or more,
    the filter has an aperture ratio of 30% or more and 50% or less,
    the fibers have a diameter of 10 μm or more and 40 μm or less, and
    the weave structure is a plain weave or a 2/1 twill weave.

2. The blood filtration filter according to claim 1, wherein the filter has OPs (openings) with a standard deviation of less than 3 μm.

3. The blood filtration filter according to claim 1, wherein the filter is formed of monofilament fibers.

4. The blood filtration filter according to claim 1, wherein OPs (openings) of the filter are 20 $\mu$m or more and 50 $\mu$m or less.

5. A blood filtration filter component, comprising the blood filtration filter according to any one of claims 1 to 4 and a supporting member holding the filter.

## INTERNATIONAL SEARCH REPORT

| International application No. |
|---|
| **PCT/JP2023/035892** |

| A. | CLASSIFICATION OF SUBJECT MATTER |
|---|---|

*A61M 1/36*(2006.01)i
FI: A61M1/36 119

According to International Patent Classification (IPC) or to both national classification and IPC

| B. | FIELDS SEARCHED |
|---|---|

Minimum documentation searched (classification system followed by classification symbols)

A61M1/36

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2019/044449 A1 (TORAY INDUSTRIES, INC.) 07 March 2019 (2019-03-07) paragraphs [0002], [0008], [0034]-[0055], tables 1-3, fig. 1 | 1-5 |
| A | WO 88/02264 A1 (TERUMO KK) 07 April 1988 (1988-04-07) entire text, all drawings | 1-5 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **13 October 2023** | **07 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/035892**

| Patent document cited in search report | | | Publication date (day/month/year) | Patent family member(s) | | | Publication date (day/month/year) |
|---|---|---|---|---|---|---|---|
| WO | 2019/044449 | A1 | 07 March 2019 | EP | 3677709 | A1 | |
| | | | | paragraphs [0002], [0008], [0030]-[0048], tables 1-3, fig. 1 | | | |
| | | | | CN | 110770376 | A | |
| WO | 88/02264 | A1 | 07 April 1988 | EP | 365676 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004008693 A
- JP 2016052525 A